(19) Europäisches Patentamt / European Patent Office / Office européen des brevets

(11) EP 2 800 607 B1

(12) EUROPÄISCHE PATENTSCHRIFT

(45) Veröffentlichungstag und Bekanntmachung des Hinweises auf die Patenterteilung:
**31.01.2018 Patentblatt 2018/05**

(21) Anmeldenummer: **12784253.2**

(22) Anmeldetag: **09.11.2012**

(51) Int Cl.:
***A61Q 5/06** (2006.01)* ***A61K 8/81** (2006.01)*

(86) Internationale Anmeldenummer:
**PCT/EP2012/072282**

(87) Internationale Veröffentlichungsnummer:
**WO 2013/091997 (27.06.2013 Gazette 2013/26)**

# (54) MITTEL ZUR TEMPORÄREN VERFORMUNG KERATINISCHER FASERN AUF GRUNDLAGE EINER KOMBINATION SPEZIFISCHER FILMBILDENDER POLYMERE

PRODUCT FOR TEMPORARILY SHAPING KERATIN FIBERS ON THE BASIS OF A COMBINATION OF SPECIFIC FILM-FORMING POLYMERS

PRODUIT DE MISE EN FORME TEMPORAIRE DE FIBRES KÉRATINIQUES À BASE D'UNE ASSOCIATION DE POLYMÈRES FILMOGÈNES SPÉCIFIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **20.12.2011 DE 102011089168**

(43) Veröffentlichungstag der Anmeldung:
**12.11.2014 Patentblatt 2014/46**

(73) Patentinhaber: **Henkel AG & Co. KGaA**
**40589 Düsseldorf (DE)**

(72) Erfinder:
- **METTEN, Diane**
  **22393 Hamburg (DE)**
- **RICHTERS, Bernd**
  **21129 Hamburg (DE)**
- **SCHEFFLER, Rene**
  **25470 Ellerau (DE)**

(56) Entgegenhaltungen:
**EP-A1- 2 272 500 DE-A1- 10 245 586**

- **Nalco: "Fixomer Anionic Polymers for Personal Care", US , 1. April 2006 (2006-04-01), XP002728590, Gefunden im Internet: URL:https://www.in-cosmetics.com/__novadocuments/2727 [gefunden am 2014-08-18]**
- **HÖSSEL P ET AL: "Polyquaternium-68: Mehr als Haarfestigung", SOFW-JOURNAL( SEIFEN,OELE,FETTE,WACHSE ), VERLAG F}R CHEMISCHE INDUSTRIE, AUGSBURG, DE , Bd. 131 1. Mai 2005 (2005-05-01), Seiten 19-29, XP002540039, Gefunden im Internet: URL:http://www.sofw.com/index/sofw_de/sofw_de_archiv.html?cosearch=polyquaternium-68&cosearch_sa=polyquaternium-68&costart=21&date_from[Y]=2005&date_from[m]=4&date_until[Y]=2005&date_until[m]=7&do_search_sa=1&naid=604 [gefunden am 2009-08-05]**
- **"LUVIQUAT POLYMER GRADES", INTERNET CITATION, November 2005 (2005-11), XP002460056, Gefunden im Internet: URL:http://www.cosmetics.basf.de/pdf/statements/Technical%20Information/Cosmetic%20Ingredients/Luviquat%20Polymer.pdf [gefunden am 2007-11-27]**
- **DATABASE GNPD [Online] MINTEL; 1. August 2009 (2009-08-01), DEP Indústria Comércio de Cosméticos: "Glue Styling Gel", XP002728591, Database accession no. 1157210**

EP 2 800 607 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die vorliegende Anmeldung beschreibt kosmetische Mittel auf Grundlage einer spezifischen Polymerkombination, die Verwendung dieser kosmetischen Mittel zur temporären Verformung keratinischer Fasern sowie kosmetische Verfahren unter Einsatz dieser Mittel.

Der Einsatz von Polymeren in verschiedensten kosmetischen Mitteln ist weit verbreitet. Sie finden sich in Mitteln zur Behandlung der Haut ebenso wie in Mitteln zur Behandlung der Haare, in Mitteln, die unmittelbar nach der Anwendung wieder ab- bzw. ausgewaschen werden, so genannten Rinse-off Produkten, genauso wie in Mitteln, die auf Haut oder Haar verbleiben, so genannten Leave-on Mitteln. Dabei werden die Polymere aus unterschiedlichsten Gründen eingesetzt und jeweils bestimmte Eigenschaften der Polymere ausgenutzt. In Mitteln zur Hautbehandlung, in Shampoos, Haarspülungen und Haarkuren stehen oftmals die verdickenden oder pflegenden Eigenschaften der Polymere im Vordergrund. In Mitteln zur temporären Verformung keratinischer Fasern, im Folgenden auch Stylingmittel genannt, sind neben diesen Eigenschaften vor allem filmbildende und/oder festigende Wirkungen gefragt. Oftmals dienen Polymere auch als Hilfsmittel um die Abscheidung und Fixierung anderer Wirk- und Inhaltsstoffe auf der Haut oder dem Haar zu verbessern oder erst möglich zu machen. So lassen sich beispielsweise durch Zusatz geeigneter Polymere zu Haarfärbemitteln die Reibechtheiten und die Beständigkeit der Färbung erhöhen.

In der Regel enthalten kosmetische Mittel einzelne Polymere, die speziell darauf zugeschnitten sind, einen ganz bestimmten Effekt zu erzielen. Sollen verschiedene Effekte erzielt werden, ist die Zugabe mehrerer Polymere erforderlich. Werden allerdings zu viele verschiedene Polymere eingesetzt, kann das eine Reihe von Nachteilen mit sich bringen. So können Probleme bei der Formulierung entstehen, etwa weil die Polymere untereinander oder mit anderen Bestandteilen des Mittels reagieren und es zu Ausfällungen oder Zersetzungen kommt. Bestimmte Polymere neigen auch dazu, sich auf der Haut und insbesondere auf dem Haar so beständig abzuscheiden, dass sie bei einer gewöhnlichen Wäsche nicht mehr vollständig entfernt werden und es zu einer unerwünschten Anreicherung des Polymers und damit letztlich einer Belastung von Haut oder Haar kommt.

Es besteht daher ständig Bedarf an Polymeren oder geeigneten Kombinationen weniger Polymere, die möglichst viele der gewünschten Eigenschaften gleichzeitig aufweisen.

Beispielsweise ist es im Falle der Stylingmittel notwendig, dass die eingesetzten Polymeren dem behandelten Haar einen möglichst starken Halt geben. Neben einem hohen Haltegrad müssen Stylingmittel jedoch eine ganze Reihe weiterer Anforderungen erfüllen. Diese können grob in Eigenschaften am Haar, Eigenschaften der jeweiligen Formulierung, z.B. Eigenschaften des Schaums, des Gels oder des versprühten Aerosols, und Eigenschaften, die die Handhabung des Stylingmittels betreffen, unterteilt werden, wobei den Eigenschaften am Haar besondere Wichtigkeit zukommt. Zu nennen sind insbesondere Feuchtebeständigkeit, niedrige Klebrigkeit und ein ausgewogener Konditioniereffekt. Weiterhin soll ein Stylingmittel möglichst für alle Haartypen universell einsetzbar sein. Handelt es sich bei dem Stylingmittel um ein Gel oder eine Paste, sollen die Polymere zudem verdickende Eigenschaften besitzen.

Die Aufgabe der vorliegenden Erfindung war es daher, weitere geeignete Polymerkombinationen zur Verfügung zu stellen, welche sich durch gute filmbildende und/oder festigende Eigenschaften auszeichnen, einen sehr hohen Haltegrad besitzen ohne dass dabei auf Flexibilität und gute Feuchtebeständigkeit - insbesondere Schweiß- und Wasserbeständigkeit - verzichtet werden müsste und sich zudem für die Herstellung stabil viskoser sowie stabil transparenter kosmetischer Zusammensetzungen eignen.

Diese Aufgaben wurden durch eine spezielle Polymerkombination gelöst. Ein erster Gegenstand der Erfindung ist daher ein kosmetisches Mittel, enthaltend in einem kosmetisch akzeptablen Träger

a) mindestens ein Copolymer A der 2-Acrylamido-2-methylpropansulfonsäure mit einem oder mehreren Monomeren ausgewählt aus Acrylsäure und Methacrylsäure,
b) mindestens eine polymere quartäre Ammoniumverbindung aus der Gruppe der Copolymere B von Vinylpyrrolidon mit Methacrylamidopropyltrimethylammoniumchlorid.

Die europäische Patentanmeldung EP 2 272 500 A1 beschreibt kosmetische Mittel zur temporären Verformung keratinischer Fasern, welches neben weiteren Bestandteilen ein Copolymer von Methacrylsäure und 2-Acrylamido-2-methylpropansulfonsäure sowie ein Copolymer von Vinylpyrrolidon, Vinylcaprolactam und N-Methylvinylimidazol enthält.

Die erfindungsgemäßen Mittel enthalten die Wirkstoffe in einem kosmetischen Träger. Dieser kosmetische Träger ist im Sinne der Erfindung ist wässrig, alkoholisch oder wässrig-alkoholisch.

Unter wässrig-alkoholischen Trägern sind im Sinne der vorliegenden Erfindung wasserhaltige Zusammensetzungen, enthaltend 3 bis 70 Gew.-% eines $C_1$-$C_4$-Alkohols, bezogen auf das Gesamtgewicht der Anwendungsmischung, insbesondere Ethanol bzw. Isopropanol, zu verstehen. Ein wässriger Träger enthält im Sinne der Erfindung mindestens 30 Gew.-%, insbesondere mindestens 50 Gew.-% Wasser, bezogen auf das Gesamtgewicht der Anwendungsmischung. Bevorzugte kosmetische Mittel enthalten, bezogen auf sein Gesamtgewicht, 40 bis 99 Gew.-%, vorzugsweise 50 bis 98 Gew.-%, besonders bevorzugt 60 bis 95 Gew.-% und insbesondere 70 bis 90 Gew.-% Wasser. Der pH-Wert (10%-ige

Lösung, 20°C) bevorzugter kosmetischer Mittel beträgt 4 bis 9, vorzugsweise 5 bis 8 und insbesondere von 6 bis 7. Als ersten wesentlichen Bestandteil enthalten die erfindungsgemäßen kosmetischen Mittel mindestens ein Copolymer A der 2-Acrylamido-2-methylpropansulfonsäure (AMPS) mit einem oder mehreren Monomeren ausgewählt aus Acrylsäure und Methacrylsäure.

Bevorzugte erfindungsgemäße Mittel enthalten mindestens ein AMPS Copolymer, ausgewählt aus

a1) Copolymeren von Acrylsäure mit 2-Acrylamido-2-methylpropansulfonsäure
a2) Copolymeren von Methacrylsäure mit 2-Acrylamido-2-methylpropansulfonsäure
a3) Copolymeren von Acrylsäure mit Methacrylsäure und 2-Acrylamido-2-methylpropansulfonsäure
a4) Copolymeren von Acrylsäureestern mit 2-Acrylamido-2-methylpropansulfonsäure
a5) Copolymeren von Methacrylsäureestern mit 2-Acrylamido-2-methylpropansulfonsäure.

Als für die kosmetische Wirkung erfindungsgemäßer Mitte besonders vorteilhaft hat sich der Einsatz von Methacrylsäure/2-Acrylamido-2-methylpropansulfonsäure Copolymeren erwiesen.

Diese lassen sich durch die allgemeine Formel

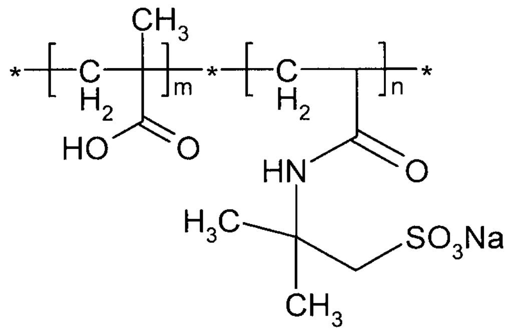

beschreiben, wobei die Indices m und n je nach Molmasse des Polymers variieren und nicht bedeuten sollen, daß es sich um Blockcopolymere handelt. Vielmehr können Struktureinheiten im Molekül statistisch verteilt vorliegen.

Besonders bevorzugte erfindungsgemäße Mittel sind dadurch gekennzeichnet, daß sie als Copolymer A Copolymere von Methacrylsäure mit 2-Acrylamido-2-methylpropansulfonsäure mit einer Molmasse von 100 bis 2500 kDa, vorzugsweise von 250 bis 2000 kDa, weiter bevorzugt von 500 bis 1750 kDa und insbesondere von 800 bis 1500 kDa, enthalten.

Copolymere von Methacrylsäure und 2-Acrylamido-2-methylpropansulfonsäure sind beispielsweise unter dem Handelsnamen Fixomer® A-30 (Nalco) erhältlich.
Besonders bevorzugt werden daher kosmetische Mittel, bei denen das Copolymer A ausgewählt ist aus der Gruppe der Copolymere von Methacrylsäure mit 2-Acrylamido-2-methylpropansulfonsäure. Besonders bevorzugt werden insbesondere solche Copolymere A, die zu mindestens 70 Gew.-%, vorzugsweise zu mindestens 80 Gew.-%, bevorzugt zu mindestens 90 Gew.-% und insbesondere zu mindestens 95 Gew.-% auf Acrylsäure, Methacrylsäure und 2-Acrylamido-2-methylpropansulfonsäure basieren. Ganz besonders bevorzugt werden insbesondere solche Copolymere A, die zu mindestens 70 Gew.-%, vorzugsweise zu mindestens 80 Gew.-%, bevorzugt zu mindestens 90 Gew.-% und insbesondere zu mindestens 95 Gew.-% auf Methacrylsäure und 2-Acrylamido-2-methylpropansulfonsäure basieren.

Der Gewichtsanteil des Copolymers A am Gesamtgewicht erfindungsgemäßer kosmetischer Mittel beträgt bevorzugt 0,05 bis 10 Gew.-%, vorzugsweise 0,1 bis 7,0 Gew.-% und insbesondere 0,2 bis 5,0 Gew.-%.

Die erfindungsgemäßen Mittel enthaltend als zweiten wesentlichen Bestandteil eine polymere quartäre Ammoniumverbindung aus der Gruppe der Copolymere B von Vinylpyrrolidon mit Methacrylamidopropyltrimethylammoniumchlorid.

Geeignete Copoylmere B sind beispielsweise

- Vinylpyrrolidon/Methacrylamidopropyltrimethylammoniumchlorid Copolymer (INCI-Bezeichnung Polyquaternium-28), beispielsweise das Copoylmer Gafquat® HS 100 (ISP).

Die erfindungsgemäßen Mittel enthalten mindestens ein Copolymer B, ausgewählt aus

b1) Copolymeren von Vinylpyrrolidon mit Methacrylamidopropyltrimethylammoniumchlorid (MAPTAC).

So sind beispielsweise kosmetische Mittel bevorzugt, die als Copolymer B Copolymere von Vinylpyrrolidon mit

Methacrylamidopropyltrimethylammoniumchlorid (MAPTAC) enthalten (b1).

Diese lassen sich durch die allgemeine Formel

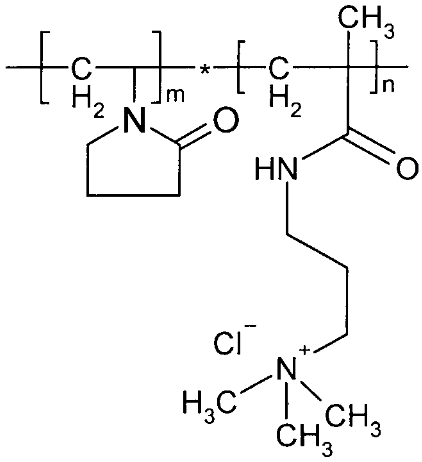

beschreiben, wobei die Indices m und n je nach Molmasse des Polymers variieren und nicht bedeuten sollen, dass es sich um Blockcopolymere handelt. Vielmehr können Struktureinheiten im Molekül statistisch verteilt vorliegen.

Besonders bevorzugte kosmetische Mittel sind dadurch gekennzeichnet, dass sie als kationisches Polymer b1 Copolymere von Methacrylamidopropyltrimethylammoniumchlorid (MAPTAC) mit Vinylpyrrolidon enthalten, die 40 bis 95 Mol.-%, vorzugsweise 42,5 bis 90 Mol.-%, weiter bevorzugt 45 bis 85 Mol.-% und insbesondere 50 bis 80 Mol.-% Vinylpyrrolidon enthalten.

Besonders bevorzugte kosmetische Mittel sind weiter dadurch gekennzeichnet, dass die Copolymere b1 Molmassen von 10 bis 1000 kDa, vorzugsweise von 25 bis 900 kDa, weiter bevorzugt von 50 bis 800 kDa und insbesondere von 100 bis 750 kDa, aufweisen.

Ein ganz besonders bevorzugtes Copolymer b1 wird nach der INCI-Nomenklatur als Polyquaternium-28 bezeichnet. Ein solches Polymer ist beispielsweise unter der Handelsbezeichnung Gafquat® HS-100 (ISP) erhältlich.

Zusätzlich zu dem bzw. den Polymer(en) b1 können die kosmetischen Mittel auch Polymere b2 aus der Gruppe der Copolymeren von Vinylpyrrolidon mit Dimethylaminoethylmethacrylat enthalten.

Diese lassen sich durch die allgemeine Formel

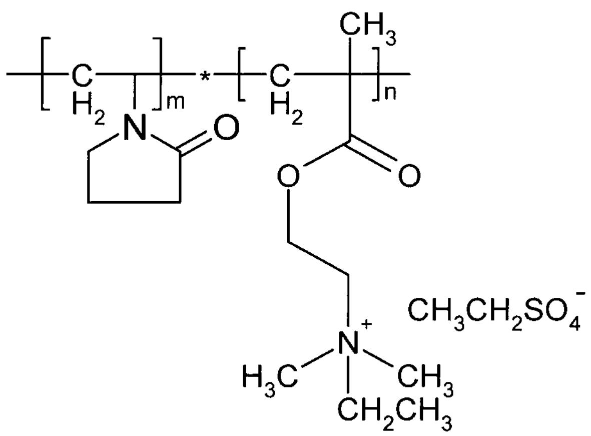

beschreiben, wobei die Indices m und n je nach Molmasse des Polymers variieren und nicht bedeuten sollen, dass es sich um Blockcopolymere handelt. Vielmehr können Struktureinheiten im Molekül statistisch verteilt vorliegen.

Besonders bevorzugte kosmetische Mittel sind dadurch gekennzeichnet, dass sie als kationisches Polymer b2 Copolymere von Vinylpyrrolidon mit Dimethylaminoethylmethacrylat enthält, die 40 bis 95 Mol.-%, vorzugsweise 42,5 bis 90 Mol.-%, weiter bevorzugt 45 bis 85 Mol.-% und insbesondere 50 bis 80 Mol.-% Vinylpyrrolidon enthalten.

Besonders bevorzugte kosmetische Mittel sind weiter dadurch gekennzeichnet, dass die Copolymere b2 Molmassen von 100 bis 2500 kDa, vorzugsweise von 250 bis 2000 kDa, weiter bevorzugt von 500 bis 1750 kDa und insbesondere von 800 bis 1500 kDa, aufweisen.

Ein ganz besonders bevorzugtes Copolymer b2 wird nach der INCI-Nomenklatur als Polyquaternium-11 bezeichnet. Ein solches Polymer ist beispielsweise unter der Handelsbezeichnung Gafquat® 755 N (ISP) erhältlich.

Zusätzlich zu dem bzw. den Polymer(en) b1 können die erfindungsgemäßen kosmetischen Mittel auch Polymere b3 aus der Gruppe der Copolymeren von Vinylpyrrolidon mit Dimethylaminopropylmethacrylamid und Alkyldimethylpropylmethacrylamidoammoniumsalzen enthalten.

Diese lassen sich durch die allgemeine Formel

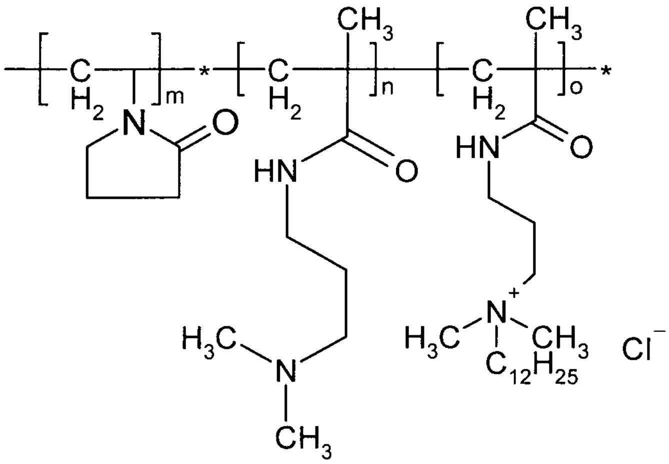

beschreiben, wobei die Indices m, n und o je nach Molmasse des Polymers variieren und nicht bedeuten sollen, dass es sich um Blockcopolymere handelt. Vielmehr können Struktureinheiten im Molekül statistisch verteilt vorliegen.

Besonders bevorzugte kosmetische Mittel sind dadurch gekennzeichnet, dass sie als kationisches Polymer b3 Copolymere von Vinylpyrrolidon mit Dimethylaminopropylmethacrylamid und Lauryldimethylpropylmethacrylamidoammoniumsalzen enthalten.

Besonders bevorzugte kosmetische Mittel sind weiter dadurch gekennzeichnet, dass sie als kationisches Polymer b3 Copolymere von Vinylpyrrolidon mit Dimethylaminopropylmethacrylamid und Alkyldimethylpropylmethacrylamidoammoniumsalzen enthalten, die 40 bis 95 Mol.-%, vorzugsweise 42,5 bis 90 Mol.-%, weiter bevorzugt 45 bis 85 Mol.-% und insbesondere 50 bis 80 Mol.-% Vinylpyrrolidon enthalten.

Ganz besonders bevorzugte kosmetische Mittel sind darüber hinaus dadurch gekennzeichnet, dass die Copolymere b3 Molmassen von 10 bis 1000 kDa, vorzugsweise von 25 bis 900 kDa, weiter bevorzugt von 50 bis 800 kDa und insbesondere von 100 bis 750 kDa, aufweisen

Ein ganz besonders bevorzugtes Copolymer b3 wird nach der INCI-Nomenklatur als Polyquaternium-55 bezeichnet. Ein solches Polymer ist beispielsweise unter der Handelsbezeichnung Styleze® W20 (ISP) erhältlich.

Als für die kosmetische Wirkung erfindungsgemäßer Mitte besonders vorteilhaft hat sich der Einsatz von Vinylpyrrolidon/Methacrylamidopropyltrimethylammoniumchlorid Copolymeren erwiesen. Besonders bevorzugt werden insbesondere solche Copolymere B, die zu mindestens 70 Gew.-%, vorzugsweise zu mindestens 80 Gew.-%, bevorzugt zu mindestens 90 Gew.-% und insbesondere zu mindestens 95 Gew.-% auf Vinylpyrrolidon und Methacrylamidopropyltrimethylammoniumchlorid basieren.

Der Gewichtsanteil des Copolymers B am Gesamtgewicht erfindungsgemäßer kosmetischer Mittel beträgt bevorzugt 0,05 bis 10 Gew.-%, vorzugsweise 0,1 bis 7,0 Gew.-% und insbesondere 0,2 bis 5,0 Gew.-%.

Die erfindungsgemäßen Mittel zeichnen sich von kosmetischen Mitteln mit alternativen Vinylpyrrolidon Copolymeren neben den oben genannten Vorteilen insbesondere auch durch einen verbesserten Haltegrad aus. Als für die kosmetischen Eigenschaften der erfindungsgemäßen Mittel besonders vorteilhaft hat sich ein Gewichtsverhältnis der Polymere A und B in dem kosmetischen Mittel zwischen 8:1 und 1:8, vorzugsweise zwischen 6:1 und 1:6 und insbesondere zwischen 4:1 und 1:4 erwiesen.

Das Copolymer A wird in dem kosmetischen Mitteln vorzugsweise in teilneutraliserter oder neutralisierter Form eingesetzt. Zur Neutralisation wird bevorzugt mindestens ein Alkanolamin verwendet. Die als erfindungsgemäßes Alkalisierungsmittel einsetzbaren Alkanolamine werden bevorzugt ausgewählt aus primären Aminen mit einem $C_2$-$C_6$-Alkylgrundkörper, der mindestens eine Hydroxylgruppe trägt. Besonders bevorzugte Alkanolamine werden aus der Gruppe ausgewählt, die gebildet wird, aus 2-Aminoethan-1-ol (Monoethanolamin), Tris(2-hydroxyethyl)-amin (Triethanolamin), 3-Aminopropan-1-ol, 4-Aminobutan-1-ol, 5-Aminopentan-1-ol,-1-Aminopropan-2-ol, 1-Aminobutan-2-ol, 1-Aminopentan-2-ol, 1-Aminopentan-3-ol, 1-Aminopentan-4-ol, 3-Amino-2-methylpropan-1-ol, 1-Amino-2-methylpropan-2-ol, 3-Aminopropan-1,2-diol, 2-Amino-2-methylpropan-1,3-diol. Erfindungsgemäß ganz besonders bevorzugte Alkanolamine werden ausgewählt aus der Gruppe 2-Aminoethan-1-ol, 2-Amino-2-methylpropan-1-ol und 2-Amino-2-methyl-propan-1,3-diol. Als besonders geeignetes Neutralisationsmittel hat sich dabei 2-Amino-2-methylpropanol erwiesen. Erfindungsgemäß bevorzugte kosmetische Mittel enthalten mindestens ein Alkanolamin, vorzugsweise 2-Amino-2-methylpropanol. Das 2-Amino-2-methylpropanol wird in den erfindungsgemäßen Mitteln vorzugsweise in einer Menge eingesetzt, welche die zur Neutralisation des Copolymers A benötigte Menge nicht überschreitet. Vorzugsweise beträgt die in den erfindungsgemäßen Mitteln eingesetzte Mengen an 2-Amino-2-methylpropanol 80 bis 100%, besonders bevorzugt 90 bis 100% und insbesondere 95 bis 100% der zur vollständigen Neutralisation des Copolymers A benötigten Menge. In einer bevorzugten Ausführungsform beträgt der Gewichtsanteil des 2-Amino-2-methylpropanols am Gesamtgewicht des kosmetischen Mittels 0,1 bis 4,0 Gew.-%, bevorzugt 0,2 bis 3,0 Gew.% und insbesondere 0,5 bis 2,0 Gew.-%.

Neben den zuvor beschriebenen Copolymeren und Trägersubstanzen können die erfindungsgemäßen kosmetischen Mittel weitere Inhaltsstoffe enthalten. Zur Gruppe dieser weiteren Inhaltsstoffe zählen insbesondere die kosmetisch wirksamen Hilfs- und Zusatzstoffe.

Als bevorzugten Bestandteil enthalten die erfindungsgemäßen kosmetischen Mittel mindestens eine quartäre Ammoniumverbindung. Als quartäre Ammoniumverbindung können monomere oder polymere Wirkstoffe eingesetzt werden.

Aus der Vielzahl an möglichen monomeren quartären Ammoniumverbindungen haben sich die Verbindungen aus den Gruppen:

- Trimethylalkylammoniumhalogenide;
- der Esterquats
- der quartären Imidazoline

als besonders wirkungsvoll erwiesen.

Zur Gruppe der Trimethylalkylammoniumhalogenide zählen insbesondere die Verbindungen der Formel (Tkat1-1).

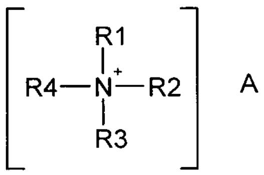

(Tkat1)

In der Formel (Tkat1) stehen R1, R2, R3 und R4 für jeweils unabhängig voneinander für Wasserstoff, eine Methylgruppe, eine Phenylgruppe, eine Benzylgruppe, für einen gesättigten, verzweigten oder unverzweigten Alkylrest mit einer Kettenlänge von 8 bis 30 Kohlenstoffatomen, welcher gegebenenfalls mit einer oder mehreren Hydroxygruppen substituiert sein kann. A steht für ein physiologisch verträgliches Anion, beispielsweise Halogenide wie Chlorid oder Bromid sowie Methosulfate.

Beispiele für Verbindungen der Formel (Tkat1) sind Lauryltrimehtylammoniumchlorid, Cetyltrimethylammoniumchlorid, Cetyltrimethylammoniumbromid, Cetyltrimethylammoniummethosulfat, Dicetyldimethylammoniumchlorid, Tricetylmethylammoniumchlorid, Stearyltrimethylammoniumchlorid, Distearyldimethylammoniumchlorid, Lauryldimethylbenzylammoniumchlorid, Behenyltrimethylammoniumchlorid, Behenyltrimethylammoniumbromid, Behenyltrimethylammoniummethosulfat. Bevorzugte kosmetische Mittel enthalten eine monomere quartäre Ammoniumverbindung aus der Gruppe der Trimethylalkylammoniumhalogenide.

Weitere erfindungsgemäß besonders bevorzugte quartären Ammoniumverbindungen sind die kationischen Betainestern der Formel (Tkat1-2.1).

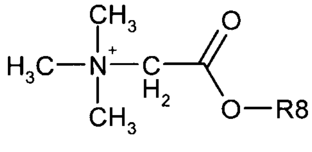

(Tkat1-2.1)

Besonders bevorzugt sind die Esterquats mit den Handelsbezeichnungen Armocare® VGH-70, sowie Dehyquart® F-75, Dehyquart® L80, Stepantex® VS 90 und Akypoquat® 131.

Eine weitere Gruppe sind quartäre Imidazolinverbindungen. Die im Folgenden dargestellte Formel (Tkat2) zeigt die Struktur dieser Verbindungen.

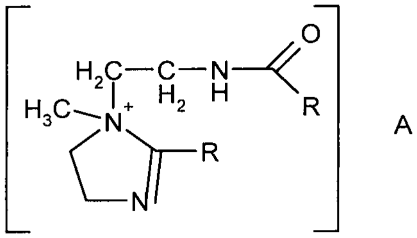

(Tkat2)

Die Reste R stehen unabhängig voneinander jeweils für einen gesättigten oder ungesättigten, linearen oder verzweigten Kohlenwasserstoffrest mit einer Kettenlänge von 8 bis 30 Kohlenstoffatomen. Die bevorzugten Verbindungen der Formel (Tkat2) enthalten für R jeweils den gleichen Kohlenwasserstoffrest. Die Kettenlänge der Reste R beträgt bevorzugt 12 bis 21 Kohlenstoffatome. A steht für ein Anion wie zuvor beschrieben. Besonders erfindungsgemäße Beispiele sind beispielsweise unter den INCI - Bezeichnungen Quaternium-27, Quaternium-72, Quaternium-83, Quaternium-87 und Quaternium-91 erhältlich. Höchst bevorzugt ist erfindungsgemäß Quaternium-91.

In Bezug auf die kosmetische Wirkung haben sich kosmetische Mittel als vorteilhaft erwiesen, bei denen der Gewichtsanteil der monomeren quartären Ammoniumverbindung am Gesamtgewicht des Mittels 0,05 bis 3,0 Gew.-%, vorzugsweise 0,1 bis 2,0 Gew.-% und insbesondere 0,2 bis 1,0 Gew.-% beträgt.

Als geeignete Hilfs- und Zusatzstoffe sind insbesondere zusätzliche Pflegestoffe zu nennen.

Als Pflegestoff einer anderen Verbindungsklasse kann das Mittel beispielsweise mindestens ein Proteinhydrolysat und/oder eines seiner Derivate enthalten. Proteinhydrolysate sind Produktgemische, die durch sauer, basisch oder enzymatisch katalysierten Abbau von Proteinen (Eiweißen) erhalten werden. Unter dem Begriff Proteinhydrolysate werden erfindungsgemäß auch Totalhydrolysate sowie einzelne Aminosäuren und deren Derivate sowie Gemische aus verschiedenen Aminosäuren verstanden. Das Molgewicht der erfindungsgemäß einsetzbaren Proteinhydrolysate liegt zwischen 75, dem Molgewicht für Glycin, und 200.000, bevorzugt beträgt das Molgewicht 75 bis 50.000 und ganz besonders bevorzugt 75 bis 20.000 Dalton.

Als Pflegestoff kann das erfindungsgemäße Mittel weiterhin mindestens ein Vitamin, ein Provitamin, eine Vitaminvorstufe und/oder eines derer Derivate enthalten. Dabei sind erfindungsgemäß solche Vitamine, Provitamine und Vitaminvorstufen bevorzugt, die üblicherweise den Gruppen A, B, C, E, F und H zugeordnet werden.

Wie auch der Zusatz von Glycerin und/oder Propylenglykol erhöht der Zusatz von Panthenol die Flexibilität des bei Anwendung des erfindungsgemäßen Mittels gebildeten Polymerfilms.

Als Pflegestoff können die erfindungsgemäßen Mittel weiterhin mindestens einen Pflanzenextrakt, aber auch Mono- bzw. Oligosaccharide und/oder Lipide enthalten.

Weiterhin sind als Pflegestoff Ölkörper geeignet. Zu den natürlichen und synthetischen kosmetischen Ölkörpern sind beispielsweise zu zählen pflanzliche Öle, flüssige Paraffinöle, Isoparaffinöle und synthetische Kohlenwasserstoffe sowie Di-n-alkylether mit insgesamt zwischen 12 bis 36 C-Atomen, insbesondere 12 bis 24 C-Atomen. Bevorzugte erfindungsgemäße kosmetische Mittel enthalten mindestens einen Ölkörper, vorzugsweise mindestens einen Ölkörper aus der Gruppe der Silikonöle. Zur Gruppe der Silikonöle zählen insbesondere die Dimethicone, zu welchen auch die Cyclomethicone zu rechnen sind, die aminofunktionellen Silikone sowie die Dimethiconole. Die Dimethicone können sowohl linear als auch verzweigt als auch cyclisch oder cyclisch und verzweigt sein. Geeignete Silikonöle oder Silikongums sind insbesondere Dialkyl- und Alkylarylsiloxane, wie beispielsweise Dimethylpolysiloxan und Methylphenylpolysiloxan, sowie deren alkoxylierte, quaternierte oder auch anionische Derivate. Bevorzugt sind cyclische und lineare Polydialkylsiloxane, deren alkoxylierte und/oder aminierte Derivate, Dihydroxypolydimethylsiloxane und Polyphenylalkylsiloxane.

Esteröle, das heißt Ester von $C_6$ - $C_{30}$ - Fettsäuren mit $C_2$ - $C_{30}$ - Fettalkoholen, vorzugsweise Monoester der Fettsäuren mit Alkoholen mit 2 bis 24 C-Atomen wie beispielsweise Isopropylmyristat (Rilanit® IPM), Isononansäure-C16-18-alkylester (Cetiol® SN), 2-Ethylhexylpalmitat (Cegesoft® 24), Stearinsäure-2-ethylhexylester (Cetiol® 868), Cetyloleat, Glycerintricaprylat, Kokosfettalkohol-caprinat/-caprylat (Cetiol® LC), n-Butylstearat, Oleylerucat (Cetiol® J 600), Isopropylpalmitat (Rilanit® IPP), Oleyl Oleate (Cetiol®), Laurinsäurehexylester (Cetiol® A), Di-n-butyladipat (Cetiol® B), Myristylmyristat (Cetiol® MM), Cetearyl Isononanoate (Cetiol® SN), Ölsäuredecylester (Cetiol® V) sind weitere bevorzugte pflegende Ölkörper.

Als Pflegestoffe eignen sich weiterhin Dicarbonsäureester, symmetrische, unsymmetrische oder cyclische Ester der Kohlensäure mit Fettalkoholen, Trifettsäureester von gesättigten und/oder ungesättigten linearen und/oder verzweigten Fettsäuren mit Glycerin oder Fettsäurepartialglyceride, worunter Monoglyceride, Diglyceride und deren technische Gemische zu verstehen sind.

In Bezug auf die kosmetische Wirkung haben sich kosmetische Mittel als vorteilhaft erwiesen, bei denen der Gewichtsanteil des Ölkörpers am Gesamtgewicht des Mittels 0,01 bis 5,0 Gew.-%, vorzugsweise 0,02 bis 4,0 Gew.-%

und insbesondere 0,05 bis 2,0 Gew.-% beträgt.

Die Zusammensetzung einiger bevorzugter kosmetischer Mittel kann den folgenden Tabellen entnommen werden (Angaben in Gew.-% bezogen auf das Gesamtgewicht des kosmetischen Mittels sofern nicht anders angegeben).

| | Formel 1 | Formel 2 | Formel 3 | Formel 4 | Formel 5 |
|---|---|---|---|---|---|
| Copolymer A | 0,05 bis 10 | 0,1 bis 7,0 | 0,2 bis 5,0 | 0,4 | 1,2 |
| Copolymer B | 0,05 bis 10 | 0,1 bis 7,0 | 0,2 bis 5,0 | 0,8 | 0,8 |
| Wasser, Misc | add 100 | add 100 | add 100 | add 100 | add 100 |

| | Formel 6 | Formel 7 | Formel 8 | Formel 9 | Formel 10 |
|---|---|---|---|---|---|
| Copolymer A 1) | 0,05 bis 10 | 0,1 bis 7,0 | 0,2 bis 5,0 | 0,4 | 1,2 |
| Copolymer B | 0,05 bis 10 | 0,1 bis 7,0 | 0,2 bis 5,0 | 0,8 | 0,8 |
| Wasser, Misc | add 100 | add 100 | add 100 | add 100 | add 100 |

| | Formel 11 | Formel 12 | Formel 13 | Formel 14 | Formel 15 |
|---|---|---|---|---|---|
| Copolymer A | 0,05 bis 10 | 0,1 bis 7,0 | 0,2 bis 5,0 | 0,4 | 1,2 |
| Copolymer B 2) | 0,05 bis 10 | 0,1 bis 7,0 | 0,2 bis 5,0 | 0,8 | 0,8 |
| Wasser, Misc | add 100 | add 100 | add 100 | add 100 | add 100 |

| | Formel 16 | Formel 17 | Formel 18 | Formel 19 | Formel 20 |
|---|---|---|---|---|---|
| Copolymer A 1) | 0,05 bis 10 | 0,1 bis 7,0 | 0,2 bis 5,0 | 0,4 | 1,2 |
| Copolymer B 2) | 0,05 bis 10 | 0,1 bis 7,0 | 0,2 bis 5,0 | 0,8 | 0,8 |
| Wasser, Misc | add 100 | add 100 | add 100 | add 100 | add 100 |

| | Formel 21 | Formel 22 | Formel 23 | Formel 24 | Formel 25 |
|---|---|---|---|---|---|
| Copolymer A | 0,05 bis 10 | 0,1 bis 7,0 | 0,2 bis 5,0 | 0,4 | 1,2 |
| Copolymer B | 0,05 bis 10 | 0,1 bis 7,0 | 0,2 bis 5,0 | 0,8 | 0,8 |
| Alkanolamin 3) | 80 bis 100 | 90 bis 100 | 95 bis 100 | 98 | 98 |
| Wasser, Misc | add 100 | add 100 | add 100 | add 100 | add 100 |

| | Formel 26 | Formel 27 | Formel 28 | Formel 29 | Formel 30 |
|---|---|---|---|---|---|
| Copolymer A 1) | 0,05 bis 10 | 0,1 bis 7,0 | 0,2 bis 5,0 | 0,4 | 1,2 |
| Copolymer B | 0,05 bis 10 | 0,1 bis 7,0 | 0,2 bis 5,0 | 0,8 | 0,8 |
| Alkanolamin 3) | 80 bis 100 | 90 bis 100 | 95 bis 100 | 98 | 98 |
| Wasser, Misc | add 100 | add 100 | add 100 | add 100 | add 100 |

| | Formel 31 | Formel 32 | Formel 33 | Formel 34 | Formel 35 |
|---|---|---|---|---|---|
| Copolymer A | 0,05 bis 10 | 0,1 bis 7,0 | 0,2 bis 5,0 | 0,4 | 1,2 |
| Copolymer B [2)] | 0,05 bis 10 | 0,1 bis 7,0 | 0,2 bis 5,0 | 0,8 | 0,8 |
| Alkanolamin [3)] | 80 bis 100 | 90 bis 100 | 95 bis 100 | 98 | 98 |
| Wasser, Misc | add 100 | add 100 | add 100 | add 100 | add 100 |

| | Formel 36 | Formel 37 | Formel 38 | Formel 39 | Formel 40 |
|---|---|---|---|---|---|
| Copolymer A [1)] | 0,05 bis 10 | 0,1 bis 7,0 | 0,2 bis 5,0 | 0,4 | 1,2 |
| Copolymer B [2)] | 0,05 bis 10 | 0,1 bis 7,0 | 0,2 bis 5,0 | 0,8 | 0,8 |
| Alkanolamin [3)] | 80 bis 100 | 90 bis 100 | 95 bis 100 | 98 | 98 |
| Wasser, Misc | add 100 | add 100 | add 100 | add 100 | add 100 |

| | Formel 41 | Formel 42 | Formel 43 | Formel 44 | Formel 45 |
|---|---|---|---|---|---|
| Copolymer A [1)] | 0,05 bis 10 | 0,1 bis 7,0 | 0,2 bis 5,0 | 0,4 | 1,2 |
| Copolymer B [2)] | 0,05 bis 10 | 0,1 bis 7,0 | 0,2 bis 5,0 | 0,8 | 0,8 |
| 2-Amino-2-methylpropanol [3)] | 80 bis 100 | 90 bis 100 | 95 bis 100 | 98 | 98 |
| Wasser, Misc | add 100 | add 100 | add 100 | add 100 | add 100 |

| | Formel 46 | Formel 47 | Formel 48 | Formel 49 | Formel 50 |
|---|---|---|---|---|---|
| Copolymer A [1)] | 0,05 bis 10 | 0,1 bis 7,0 | 0,2 bis 5,0 | 0,4 | 1,2 |
| Copolymer B [2)] | 0,05 bis 10 | 0,1 bis 7,0 | 0,2 bis 5,0 | 0,8 | 0,8 |
| Alkyltrimethyl-ammoniumchlorid | 0,05 bis 3,0 | 0,1 bis 2,0 | 0,2 bis 1,0 | 0,5 | 0,3 |
| Wasser, Misc | add 100 | add 100 | add 100 | add 100 | add 100 |

| | Formel 51 | Formel 52 | Formel 53 | Formel 54 | Formel 55 |
|---|---|---|---|---|---|
| Copolymer A [1)] | 0,05 bis 10 | 0,1 bis 7,0 | 0,2 bis 5,0 | 0,4 | 1,2 |
| Copolymer B [2)] | 0,05 bis 10 | 0,1 bis 7,0 | 0,2 bis 5,0 | 0,8 | 0,8 |
| Ölkörper | 0,01 bis 5,0 | 0,02 bis 4,0 | 0,05 bis 2,0 | 0,1 | 0,1 |
| Wasser, Misc | add 100 | add 100 | add 100 | add 100 | add 100 |

| | Formel 56 | Formel 57 | Formel 58 | Formel 59 | Formel 60 |
|---|---|---|---|---|---|
| Copolymer A [1)] | 0,05 bis 10 | 0,1 bis 7,0 | 0,2 bis 5,0 | 0,4 | 1,2 |
| Copolymer B [2)] | 0,05 bis 10 | 0,1 bis 7,0 | 0,2 bis 5,0 | 0,8 | 0,8 |
| Wasser | 40 bis 99 | 50 bis 98 | 60 bis 95 | 97 | 92 |
| Misc | add 100 | add 100 | add 100 | add 100 | add 100 |

| | Formel 61 | Formel 62 | Formel 63 | Formel 64 | Formel 65 |
|---|---|---|---|---|---|
| Copolymer A1) | 0,05 bis 10 | 0,1 bis 7,0 | 0,2 bis 5,0 | 0,4 | 1,2 |
| Copolymer B 2) | 0,05 bis 10 | 0,1 bis 7,0 | 0,2 bis 5,0 | 0,8 | 0,8 |
| Alkyltrimethylammoniumchlorid | 0,05 bis 3,0 | 0,1 bis 2,0 | 0,2 bis 1,0 | 0,5 | 0,3 |
| Wasser | 40 bis 99 | 50 bis 98 | 60 bis 95 | 97 | 92 |
| Misc | add 100 | add 100 | add 100 | add 100 | add 100 |

| | Formel 66 | Formel 67 | Formel 68 | Formel 69 | Formel 70 |
|---|---|---|---|---|---|
| Copolymer A 1) | 0,05 bis 10 | 0,1 bis 7,0 | 0,2 bis 5,0 | 0,4 | 1,2 |
| Copolymer B2) | 0,05 bis 10 | 0,1 bis 7,0 | 0,2 bis 5,0 | 0,8 | 0,8 |
| Ölkörper | 0,01 bis 5,0 | 0,02 bis 4,0 | 0,05 bis 2,0 | 0,1 | 0,1 |
| Wasser | 40 bis 99 | 50 bis 98 | 60 bis 95 | 97 | 92 |
| Misc | add 100 | add 100 | add 100 | add 100 | add 100 |

| | Formel 71 | Formel 72 | Formel 73 | Formel 74 | Formel 75 |
|---|---|---|---|---|---|
| Copolymer A 1) | 0,05 bis 10 | 0,1 bis 7,0 | 0,2 bis 5,0 | 0,4 | 1,2 |
| Copolymer B2) | 0,05 bis 10 | 0,1 bis 7,0 | 0,2 bis 5,0 | 0,8 | 0,8 |
| 2-Amino-2-methylpropanol 3) | 80 bis 100 | 90 bis 100 | 95 bis 100 | 98 | 98 |
| Ölkörper | 0,01 bis 5,0 | 0,02 bis 4,0 | 0,05 bis 2,0 | 0,1 | 0,1 |
| Wasser | 40 bis 99 | 50 bis 98 | 60 bis 95 | 97 | 92 |
| Misc | add 100 | add 100 | add 100 | add 100 | add 100 |

1) Copolymer A, welches zu mindestens 95 Gew.-% auf Methacrylsäure und 2-Acrylamido-2-methylpropansulfonsäure basiert.
2) Copolymer B, welches zu mindestens 95 Gew.-% auf Vinylpyrrolidon und Methacrylamidopropyltrimethylammoniumchlorid basiert
3) % der zur vollständigen Neutralisation des Copolymers A benötigten Menge

Die Formulierung der erfindungsgemäßen Mittel kann in allen für kosmetische Mittel üblichen Formen erfolgen, beispielsweise in Form von Lösungen, die beispielsweise als Haarwasser auf das Haar aufgebracht werden können, in Form von Cremes, Emulsionen, Wachsen, Gelen oder auch tensidhaltigen schäumenden Lösungen oder anderen Zubereitungen, die für die Anwendung auf dem Haar geeignet sind. In einer alternativen Ausführungsform können diese Mittel jedoch auch in Gel- oder in Cremeform vorliegen, wobei transparente Gele besonders bevorzugt sind.

Die erfindungsgemäßen Zusammensetzungen eignen sich insbesondere sehr gut dazu, Gasblasen im Mittel zu stabilisieren. Auf diese Weise können Luft oder andere Gase bzw. Gasgemische gut und langzeitstabil in die erfindungsgemäßen Mittel eingearbeitet werden. Dies kann wahlweise bei der Herstellung der Mittel geschehen, indem das Mittel vor der Abfüllung mit Gas, vorzugsweise Luft, beaufschlagt wird und ein Produkt abgefüllt wird, das sichtbare Gasblasen enthält. Bevorzugt liegen die erfindungsgemäßen Mittel in Form eines Schaums vor. Als Schaum wird dabei eine Zubereitung bezeichnet, die Gas gefüllte Bläschen aufweist, welche von flüssigen (flüssiger Schaum) oder festen (fester Schaum) Wänden umgeben sind. Bei den in der nachfolgenden Tabelle angeführten Zusammensetzungen handelt es sich vorzugsweise um feste Schäume. Die Dichte bevorzugter Zusammensetzungen beträgt 0,3 bis 1,0 g/cm$^3$, vorzugsweise 0,4 bis 0,9 g/cm$^3$ und insbesondere 0,5 bis 0,8 g/cm$^3$. Die Herstellung solcher Schäume kann beispielsweise durch Aufschlagen der Zubereitung in einem geeigneten Mischer oder durch Beaufschlagung mit einem geeigneten Gas, vorzugsweise Luft, erfolgen.

Die Zusammensetzung einiger bevorzugter kosmetischer Schäume kann der folgenden Tabellen entnommen werden. In dieser Tabelle verweist die linke Spalte ("Formel x") auf jeweils eine der in den weiter oben offenbarten

Tabellen angeführten beispielhaften kosmetischen Zusammensetzungen. Die weiteren Spalten zwei bis sieben ("Dichte") geben jeweils die Dichte der entsprechenden kosmetischen Zusammensetzung an.

Eine kosmetische Zubereitung gemäß Zeile 12, Spalte 5 der nachfolgenden Tabelle, umfasst mit anderen Worten ein kosmetisches Mittel gemäß Formel 11 mit einer Dichte von 0,44 g/cm$^3$.

| | Dichte [g/cm$^3$] | | | | | |
|---|---|---|---|---|---|---|
| Formel 1 | 0,3 bis 1,0 | 0,93 | 0,4 bis 0,9 | 0,44 | 0,5 bis 0,8 | 0,61 |
| Formel 2 | 0,3 bis 1,0 | 0,93 | 0,4 bis 0,9 | 0,44 | 0,5 bis 0,8 | 0,61 |
| Formel 3 | 0,3 bis 1,0 | 0,93 | 0,4 bis 0,9 | 0,44 | 0,5 bis 0,8 | 0,61 |
| Formel 4 | 0,3 bis 1,0 | 0,93 | 0,4 bis 0,9 | 0,44 | 0,5 bis 0,8 | 0,61 |
| Formel 5 | 0,3 bis 1,0 | 0,93 | 0,4 bis 0,9 | 0,44 | 0,5 bis 0,8 | 0,61 |
| Formel 6 | 0,3 bis 1,0 | 0,93 | 0,4 bis 0,9 | 0,44 | 0,5 bis 0,8 | 0,61 |
| Formel 7 | 0,3 bis 1,0 | 0,93 | 0,4 bis 0,9 | 0,44 | 0,5 bis 0,8 | 0,61 |
| Formel 8 | 0,3 bis 1,0 | 0,93 | 0,4 bis 0,9 | 0,44 | 0,5 bis 0,8 | 0,61 |
| Formel 9 | 0,3 bis 1,0 | 0,93 | 0,4 bis 0,9 | 0,44 | 0,5 bis 0,8 | 0,61 |
| Formel 10 | 0,3 bis 1,0 | 0,93 | 0,4 bis 0,9 | 0,44 | 0,5 bis 0,8 | 0,61 |
| Formel 11 | 0,3 bis 1,0 | 0,93 | 0,4 bis 0,9 | 0,44 | 0,5 bis 0,8 | 0,61 |
| Formel 12 | 0,3 bis 1,0 | 0,93 | 0,4 bis 0,9 | 0,44 | 0,5 bis 0,8 | 0,61 |
| Formel 13 | 0,3 bis 1,0 | 0,93 | 0,4 bis 0,9 | 0,44 | 0,5 bis 0,8 | 0,61 |
| Formel 14 | 0,3 bis 1,0 | 0,93 | 0,4 bis 0,9 | 0,44 | 0,5 bis 0,8 | 0,61 |
| Formel 15 | 0,3 bis 1,0 | 0,93 | 0,4 bis 0,9 | 0,44 | 0,5 bis 0,8 | 0,61 |
| Formel 16 | 0,3 bis 1,0 | 0,93 | 0,4 bis 0,9 | 0,44 | 0,5 bis 0,8 | 0,61 |
| Formel 17 | 0,3 bis 1,0 | 0,93 | 0,4 bis 0,9 | 0,44 | 0,5 bis 0,8 | 0,61 |
| Formel 18 | 0,3 bis 1,0 | 0,93 | 0,4 bis 0,9 | 0,44 | 0,5 bis 0,8 | 0,61 |
| Formel 19 | 0,3 bis 1,0 | 0,93 | 0,4 bis 0,9 | 0,44 | 0,5 bis 0,8 | 0,61 |
| Formel 20 | 0,3 bis 1,0 | 0,93 | 0,4 bis 0,9 | 0,44 | 0,5 bis 0,8 | 0,61 |
| Formel 21 | 0,3 bis 1,0 | 0,93 | 0,4 bis 0,9 | 0,44 | 0,5 bis 0,8 | 0,61 |
| Formel 22 | 0,3 bis 1,0 | 0,93 | 0,4 bis 0,9 | 0,44 | 0,5 bis 0,8 | 0,61 |
| Formel 23 | 0,3 bis 1,0 | 0,93 | 0,4 bis 0,9 | 0,44 | 0,5 bis 0,8 | 0,61 |
| Formel 24 | 0,3 bis 1,0 | 0,93 | 0,4 bis 0,9 | 0,44 | 0,5 bis 0,8 | 0,61 |
| Formel 25 | 0,3 bis 1,0 | 0,93 | 0,4 bis 0,9 | 0,44 | 0,5 bis 0,8 | 0,61 |
| Formel 26 | 0,3 bis 1,0 | 0,93 | 0,4 bis 0,9 | 0,44 | 0,5 bis 0,8 | 0,61 |
| Formel 27 | 0,3 bis 1,0 | 0,93 | 0,4 bis 0,9 | 0,44 | 0,5 bis 0,8 | 0,61 |
| Formel 28 | 0,3 bis 1,0 | 0,93 | 0,4 bis 0,9 | 0,44 | 0,5 bis 0,8 | 0,61 |
| Formel 29 | 0,3 bis 1,0 | 0,93 | 0,4 bis 0,9 | 0,44 | 0,5 bis 0,8 | 0,61 |
| Formel 30 | 0,3 bis 1,0 | 0,93 | 0,4 bis 0,9 | 0,44 | 0,5 bis 0,8 | 0,61 |
| Formel 31 | 0,3 bis 1,0 | 0,93 | 0,4 bis 0,9 | 0,44 | 0,5 bis 0,8 | 0,61 |
| Formel 32 | 0,3 bis 1,0 | 0,93 | 0,4 bis 0,9 | 0,44 | 0,5 bis 0,8 | 0,61 |
| Formel 33 | 0,3 bis 1,0 | 0,93 | 0,4 bis 0,9 | 0,44 | 0,5 bis 0,8 | 0,61 |
| Formel 34 | 0,3 bis 1,0 | 0,93 | 0,4 bis 0,9 | 0,44 | 0,5 bis 0,8 | 0,61 |
| Formel 35 | 0,3 bis 1,0 | 0,93 | 0,4 bis 0,9 | 0,44 | 0,5 bis 0,8 | 0,61 |

(fortgesetzt)

| | Dichte [g/cm$^3$] | | | | | |
|---|---|---|---|---|---|---|
| Formel 36 | 0,3 bis 1,0 | 0,93 | 0,4 bis 0,9 | 0,44 | 0,5 bis 0,8 | 0,61 |
| Formel 37 | 0,3 bis 1,0 | 0,93 | 0,4 bis 0,9 | 0,44 | 0,5 bis 0,8 | 0,61 |
| Formel 38 | 0,3 bis 1,0 | 0,93 | 0,4 bis 0,9 | 0,44 | 0,5 bis 0,8 | 0,61 |
| Formel 39 | 0,3 bis 1,0 | 0,93 | 0,4 bis 0,9 | 0,44 | 0,5 bis 0,8 | 0,61 |
| Formel 40 | 0,3 bis 1,0 | 0,93 | 0,4 bis 0,9 | 0,44 | 0,5 bis 0,8 | 0,61 |
| Formel 41 | 0,3 bis 1,0 | 0,93 | 0,4 bis 0,9 | 0,44 | 0,5 bis 0,8 | 0,61 |
| Formel 42 | 0,3 bis 1,0 | 0,93 | 0,4 bis 0,9 | 0,44 | 0,5 bis 0,8 | 0,61 |
| Formel 43 | 0,3 bis 1,0 | 0,93 | 0,4 bis 0,9 | 0,44 | 0,5 bis 0,8 | 0,61 |
| Formel 44 | 0,3 bis 1,0 | 0,93 | 0,4 bis 0,9 | 0,44 | 0,5 bis 0,8 | 0,61 |
| Formel 45 | 0,3 bis 1,0 | 0,93 | 0,4 bis 0,9 | 0,44 | 0,5 bis 0,8 | 0,61 |
| Formel 46 | 0,3 bis 1,0 | 0,93 | 0,4 bis 0,9 | 0,44 | 0,5 bis 0,8 | 0,61 |
| Formel 47 | 0,3 bis 1,0 | 0,93 | 0,4 bis 0,9 | 0,44 | 0,5 bis 0,8 | 0,61 |
| Formel 48 | 0,3 bis 1,0 | 0,93 | 0,4 bis 0,9 | 0,44 | 0,5 bis 0,8 | 0,61 |
| Formel 49 | 0,3 bis 1,0 | 0,93 | 0,4 bis 0,9 | 0,44 | 0,5 bis 0,8 | 0,61 |
| Formel 50 | 0,3 bis 1,0 | 0,93 | 0,4 bis 0,9 | 0,44 | 0,5 bis 0,8 | 0,61 |
| Formel 51 | 0,3 bis 1,0 | 0,93 | 0,4 bis 0,9 | 0,44 | 0,5 bis 0,8 | 0,61 |
| Formel 52 | 0,3 bis 1,0 | 0,93 | 0,4 bis 0,9 | 0,44 | 0,5 bis 0,8 | 0,61 |
| Formel 53 | 0,3 bis 1,0 | 0,93 | 0,4 bis 0,9 | 0,44 | 0,5 bis 0,8 | 0,61 |
| Formel 54 | 0,3 bis 1,0 | 0,93 | 0,4 bis 0,9 | 0,44 | 0,5 bis 0,8 | 0,61 |
| Formel 55 | 0,3 bis 1,0 | 0,93 | 0,4 bis 0,9 | 0,44 | 0,5 bis 0,8 | 0,61 |
| Formel 56 | 0,3 bis 1,0 | 0,93 | 0,4 bis 0,9 | 0,44 | 0,5 bis 0,8 | 0,61 |
| Formel 57 | 0,3 bis 1,0 | 0,93 | 0,4 bis 0,9 | 0,44 | 0,5 bis 0,8 | 0,61 |
| Formel 58 | 0,3 bis 1,0 | 0,93 | 0,4 bis 0,9 | 0,44 | 0,5 bis 0,8 | 0,61 |
| Formel 59 | 0,3 bis 1,0 | 0,93 | 0,4 bis 0,9 | 0,44 | 0,5 bis 0,8 | 0,61 |
| Formel 60 | 0,3 bis 1,0 | 0,93 | 0,4 bis 0,9 | 0,44 | 0,5 bis 0,8 | 0,61 |
| Formel 61 | 0,3 bis 1,0 | 0,93 | 0,4 bis 0,9 | 0,44 | 0,5 bis 0,8 | 0,61 |
| Formel 62 | 0,3 bis 1,0 | 0,93 | 0,4 bis 0,9 | 0,44 | 0,5 bis 0,8 | 0,61 |
| Formel 63 | 0,3 bis 1,0 | 0,93 | 0,4 bis 0,9 | 0,44 | 0,5 bis 0,8 | 0,61 |
| Formel 64 | 0,3 bis 1,0 | 0,93 | 0,4 bis 0,9 | 0,44 | 0,5 bis 0,8 | 0,61 |
| Formel 65 | 0,3 bis 1,0 | 0,93 | 0,4 bis 0,9 | 0,44 | 0,5 bis 0,8 | 0,61 |
| Formel 66 | 0,3 bis 1,0 | 0,93 | 0,4 bis 0,9 | 0,44 | 0,5 bis 0,8 | 0,61 |
| Formel 67 | 0,3 bis 1,0 | 0,93 | 0,4 bis 0,9 | 0,44 | 0,5 bis 0,8 | 0,61 |
| Formel 68 | 0,3 bis 1,0 | 0,93 | 0,4 bis 0,9 | 0,44 | 0,5 bis 0,8 | 0,61 |
| Formel 69 | 0,3 bis 1,0 | 0,93 | 0,4 bis 0,9 | 0,44 | 0,5 bis 0,8 | 0,61 |
| Formel 70 | 0,3 bis 1,0 | 0,93 | 0,4 bis 0,9 | 0,44 | 0,5 bis 0,8 | 0,61 |
| Formel 71 | 0,3 bis 1,0 | 0,93 | 0,4 bis 0,9 | 0,44 | 0,5 bis 0,8 | 0,61 |
| Formel 72 | 0,3 bis 1,0 | 0,93 | 0,4 bis 0,9 | 0,44 | 0,5 bis 0,8 | 0,61 |
| Formel 73 | 0,3 bis 1,0 | 0,93 | 0,4 bis 0,9 | 0,44 | 0,5 bis 0,8 | 0,61 |

(fortgesetzt)

| | Dichte [g/cm³] | | | | | |
|---|---|---|---|---|---|---|
| Formel 74 | 0,3 bis 1,0 | 0,93 | 0,4 bis 0,9 | 0,44 | 0,5 bis 0,8 | 0,61 |
| Formel 75 | 0,3 bis 1,0 | 0,93 | 0,4 bis 0,9 | 0,44 | 0,5 bis 0,8 | 0,61 |

In einer alternativen Ausführungsform können die erfindungsgemäßen kosmetischen Mittel als Pump- oder Aerosolspray konfektioniert werden. Bevorzugte Aerosolsprays enthalten dabei neben weiteren Aktiv- und Hilfsstoffen ein Treibmittel. Erfindungsgemäß geeignete Treibmittel (Treibgase) sind Propan, n-Butan, iso-Butan, Dimethylether (DME), Stickstoff, Luft, Lachgas, 1,1-Difluorethan, und zwar sowohl einzeln als auch in Kombination. Auch hydrophile Treibgase, wie z. B. Kohlendioxid, können vorteilhaft im Sinne der vorliegenden Erfindung eingesetzt werden, wenn der Anteil an hydrophilen Gasen gering gewählt wird und lipophiles Treibgas (z. B. Propan/Butan) im Überschuss vorliegt. Besonders bevorzugt sind Dimethylether, Propan, n-Butan, iso-Butan sowie Mischungen dieser Treibgase. Ganz besonders bevorzugt ist der Einsatz von Propan/Butan Gemischen oder Isobutan. Kosmetische Mittel, die das Treibmittel bezogen auf ihr Gesamtgewicht in einer Menge von 2,0 - 20 Gew.%, bevorzugt 4,0 - 15 Gew.% und besonders bevorzugt 5,0 - 10 Gew.-% enthalten, sind erfindungsgemäß bevorzugt.

Die Zusammensetzung einiger bevorzugter Treibmittel-haltiger kosmetischer Mittel kann der folgenden Tabellen entnommen werden. In dieser Tabelle verweist die linke Spalte ("Formel x") auf jeweils eine der in den weiter oben offenbarten Tabellen angeführten beispielhaften kosmetischen Zusammensetzungen. Die weiteren Spalten zwei bis sieben ("Treibmittel") geben jeweils die der entsprechenden kosmetischen Zusammensetzung zugesetzte Menge an Treibmittel an. Diese Angaben in "Gew.-%" beziehen sich auf das Gesamtgewicht der kosmetischen Zusammensetzung der jeweiligen "Formel x" ohne Treibmittel.

Eine kosmetische Zubereitung gemäß Zeile 12, Spalte 5 der nachfolgenden Tabelle, umfasst mit anderen Worten eine 20:1 Mischung des Treibmittel-freien kosmetischen Mittels gemäß Formel 11 mit einem Propan/Butan Gemisch.

| | Treibmittel [Gew.-%] | | | | | |
|---|---|---|---|---|---|---|
| Formel 1 | 2,0 bis 10 | 5,0 | 2,0 bis 10 P/B* | 5,0 P/B | 2,0 bis 10 iB* | 5,0 iB |
| Formel 2 | 2,0 bis 10 | 5,0 | 2,0 bis 10 P/B* | 5,0 P/B | 2,0 bis 10 iB* | 5,0 iB |
| Formel 3 | 2,0 bis 10 | 5,0 | 2,0 bis 10 P/B* | 5,0 P/B | 2,0 bis 10 iB* | 5,0 iB |
| Formel 4 | 2,0 bis 10 | 5,0 | 2,0 bis 10 P/B* | 5,0 P/B | 2,0 bis 10 iB* | 5,0 iB |
| Formel 5 | 2,0 bis 10 | 5,0 | 2,0 bis 10 P/B* | 5,0 P/B | 2,0 bis 10 iB* | 5,0 iB |
| Formel 6 | 2,0 bis 10 | 5,0 | 2,0 bis 10 P/B* | 5,0 P/B | 2,0 bis 10 iB* | 5,0 iB |
| Formel 7 | 2,0 bis 10 | 5,0 | 2,0 bis 10 P/B* | 5,0 P/B | 2,0 bis 10 iB* | 5,0 iB |
| Formel 8 | 2,0 bis 10 | 5,0 | 2,0 bis 10 P/B* | 5,0 P/B | 2,0 bis 10 iB* | 5,0 iB |
| Formel 9 | 2,0 bis 10 | 5,0 | 2,0 bis 10 P/B* | 5,0 P/B | 2,0 bis 10 iB* | 5,0 iB |
| Formel 10 | 2,0 bis 10 | 5,0 | 2,0 bis 10 P/B* | 5,0 P/B | 2,0 bis 10 iB* | 5,0 iB |
| Formel 11 | 2,0 bis 10 | 5,0 | 2,0 bis 10 P/B* | 5,0 P/B | 2,0 bis 10 iB* | 5,0 iB |
| Formel 12 | 2,0 bis 10 | 5,0 | 2,0 bis 10 P/B* | 5,0 P/B | 2,0 bis 10 iB* | 5,0 iB |
| Formel 13 | 2,0 bis 10 | 5,0 | 2,0 bis 10 P/B* | 5,0 P/B | 2,0 bis 10 iB* | 5,0 iB |
| Formel 14 | 2,0 bis 10 | 5,0 | 2,0 bis 10 P/B* | 5,0 P/B | 2,0 bis 10 iB* | 5,0 iB |
| Formel 15 | 2,0 bis 10 | 5,0 | 2,0 bis 10 P/B* | 5,0 P/B | 2,0 bis 10 iB* | 5,0 iB |
| Formel 16 | 2,0 bis 10 | 5,0 | 2,0 bis 10 P/B* | 5,0 P/B | 2,0 bis 10 iB* | 5,0 iB |
| Formel 17 | 2,0 bis 10 | 5,0 | 2,0 bis 10 P/B* | 5,0 P/B | 2,0 bis 10 iB* | 5,0 iB |
| Formel 18 | 2,0 bis 10 | 5,0 | 2,0 bis 10 P/B* | 5,0 P/B | 2,0 bis 10 iB* | 5,0 iB |
| Formel 19 | 2,0 bis 10 | 5,0 | 2,0 bis 10 P/B* | 5,0 P/B | 2,0 bis 10 iB* | 5,0 iB |
| Formel 20 | 2,0 bis 10 | 5,0 | 2,0 bis 10 P/B* | 5,0 P/B | 2,0 bis 10 iB* | 5,0 iB |

(fortgesetzt)

| | Treibmittel [Gew.-%] | | | | | |
|---|---|---|---|---|---|---|
| Formel 21 | 2,0 bis 10 | 5,0 | 2,0 bis 10 P/B* | 5,0 P/B | 2,0 bis 10 iB* | 5,0 iB |
| Formel 22 | 2,0 bis 10 | 5,0 | 2,0 bis 10 P/B* | 5,0 P/B | 2,0 bis 10 iB* | 5,0 iB |
| Formel 23 | 2,0 bis 10 | 5,0 | 2,0 bis 10 P/B* | 5,0 P/B | 2,0 bis 10 iB* | 5,0 iB |
| Formel 24 | 2,0 bis 10 | 5,0 | 2,0 bis 10 P/B* | 5,0 P/B | 2,0 bis 10 iB* | 5,0 iB |
| Formel 25 | 2,0 bis 10 | 5,0 | 2,0 bis 10 P/B* | 5,0 P/B | 2,0 bis 10 iB* | 5,0 iB |
| Formel 26 | 2,0 bis 10 | 5,0 | 2,0 bis 10 P/B* | 5,0 P/B | 2,0 bis 10 iB* | 5,0 iB |
| Formel 27 | 2,0 bis 10 | 5,0 | 2,0 bis 10 P/B* | 5,0 P/B | 2,0 bis 10 iB* | 5,0 iB |
| Formel 28 | 2,0 bis 10 | 5,0 | 2,0 bis 10 P/B* | 5,0 P/B | 2,0 bis 10 iB* | 5,0 iB |
| Formel 29 | 2,0 bis 10 | 5,0 | 2,0 bis 10 P/B* | 5,0 P/B | 2,0 bis 10 iB* | 5,0 iB |
| Formel 30 | 2,0 bis 10 | 5,0 | 2,0 bis 10 P/B* | 5,0 P/B | 2,0 bis 10 iB* | 5,0 iB |
| Formel 31 | 2,0 bis 10 | 5,0 | 2,0 bis 10 P/B* | 5,0 P/B | 2,0 bis 10 iB* | 5,0 iB |
| Formel 32 | 2,0 bis 10 | 5,0 | 2,0 bis 10 P/B* | 5,0 P/B | 2,0 bis 10 iB* | 5,0 iB |
| Formel 33 | 2,0 bis 10 | 5,0 | 2,0 bis 10 P/B* | 5,0 P/B | 2,0 bis 10 iB* | 5,0 iB |
| Formel 34 | 2,0 bis 10 | 5,0 | 2,0 bis 10 P/B* | 5,0 P/B | 2,0 bis 10 iB* | 5,0 iB |
| Formel 35 | 2,0 bis 10 | 5,0 | 2,0 bis 10 P/B* | 5,0 P/B | 2,0 bis 10 iB* | 5,0 iB |
| Formel 36 | 2,0 bis 10 | 5,0 | 2,0 bis 10 P/B* | 5,0 P/B | 2,0 bis 10 iB* | 5,0 iB |
| Formel 37 | 2,0 bis 10 | 5,0 | 2,0 bis 10 P/B* | 5,0 P/B | 2,0 bis 10 iB* | 5,0 iB |
| Formel 38 | 2,0 bis 10 | 5,0 | 2,0 bis 10 P/B* | 5,0 P/B | 2,0 bis 10 iB* | 5,0 iB |
| Formel 39 | 2,0 bis 10 | 5,0 | 2,0 bis 10 P/B* | 5,0 P/B | 2,0 bis 10 iB* | 5,0 iB |
| Formel 40 | 2,0 bis 10 | 5,0 | 2,0 bis 10 P/B* | 5,0 P/B | 2,0 bis 10 iB* | 5,0 iB |
| Formel 41 | 2,0 bis 10 | 5,0 | 2,0 bis 10 P/B* | 5,0 P/B | 2,0 bis 10 iB* | 5,0 iB |
| Formel 42 | 2,0 bis 10 | 5,0 | 2,0 bis 10 P/B* | 5,0 P/B | 2,0 bis 10 iB* | 5,0 iB |
| Formel 43 | 2,0 bis 10 | 5,0 | 2,0 bis 10 P/B* | 5,0 P/B | 2,0 bis 10 iB* | 5,0 iB |
| Formel 44 | 2,0 bis 10 | 5,0 | 2,0 bis 10 P/B* | 5,0 P/B | 2,0 bis 10 iB* | 5,0 iB |
| Formel 45 | 2,0 bis 10 | 5,0 | 2,0 bis 10 P/B* | 5,0 P/B | 2,0 bis 10 iB* | 5,0 iB |
| Formel 46 | 2,0 bis 10 | 5,0 | 2,0 bis 10 P/B* | 5,0 P/B | 2,0 bis 10 iB* | 5,0 iB |
| Formel 47 | 2,0 bis 10 | 5,0 | 2,0 bis 10 P/B* | 5,0 P/B | 2,0 bis 10 iB* | 5,0 iB |
| Formel 48 | 2,0 bis 10 | 5,0 | 2,0 bis 10 P/B* | 5,0 P/B | 2,0 bis 10 iB* | 5,0 iB |
| Formel 49 | 2,0 bis 10 | 5,0 | 2,0 bis 10 P/B* | 5,0 P/B | 2,0 bis 10 iB* | 5,0 iB |
| Formel 50 | 2,0 bis 10 | 5,0 | 2,0 bis 10 P/B* | 5,0 P/B | 2,0 bis 10 iB* | 5,0 iB |
| Formel 51 | 2,0 bis 10 | 5,0 | 2,0 bis 10 P/B* | 5,0 P/B | 2,0 bis 10 iB* | 5,0 iB |
| Formel 52 | 2,0 bis 10 | 5,0 | 2,0 bis 10 P/B* | 5,0 P/B | 2,0 bis 10 iB* | 5,0 iB |
| Formel 53 | 2,0 bis 10 | 5,0 | 2,0 bis 10 P/B* | 5,0 P/B | 2,0 bis 10 iB* | 5,0 iB |
| Formel 54 | 2,0 bis 10 | 5,0 | 2,0 bis 10 P/B* | 5,0 P/B | 2,0 bis 10 iB* | 5,0 iB |
| Formel 55 | 2,0 bis 10 | 5,0 | 2,0 bis 10 P/B* | 5,0 P/B | 2,0 bis 10 iB* | 5,0 iB |
| Formel 56 | 2,0 bis 10 | 5,0 | 2,0 bis 10 P/B* | 5,0 P/B | 2,0 bis 10 iB* | 5,0 iB |
| Formel 57 | 2,0 bis 10 | 5,0 | 2,0 bis 10 P/B* | 5,0 P/B | 2,0 bis 10 iB* | 5,0 iB |
| Formel 58 | 2,0 bis 10 | 5,0 | 2,0 bis 10 P/B* | 5,0 P/B | 2,0 bis 10 iB* | 5,0 iB |

(fortgesetzt)

| | Treibmittel [Gew.-%] | | | | | |
|---|---|---|---|---|---|---|
| Formel 59 | 2,0 bis 10 | 5,0 | 2,0 bis 10 P/B* | 5,0 P/B | 2,0 bis 10 iB* | 5,0 iB |
| Formel 60 | 2,0 bis 10 | 5,0 | 2,0 bis 10 P/B* | 5,0 P/B | 2,0 bis 10 iB* | 5,0 iB |
| Formel 61 | 2,0 bis 10 | 5,0 | 2,0 bis 10 P/B* | 5,0 P/B | 2,0 bis 10 iB* | 5,0 iB |
| Formel 62 | 2,0 bis 10 | 5,0 | 2,0 bis 10 P/B* | 5,0 P/B | 2,0 bis 10 iB* | 5,0 iB |
| Formel 63 | 2,0 bis 10 | 5,0 | 2,0 bis 10 P/B* | 5,0 P/B | 2,0 bis 10 iB* | 5,0 iB |
| Formel 64 | 2,0 bis 10 | 5,0 | 2,0 bis 10 P/B* | 5,0 P/B | 2,0 bis 10 iB* | 5,0 iB |
| Formel 65 | 2,0 bis 10 | 5,0 | 2,0 bis 10 P/B* | 5,0 P/B | 2,0 bis 10 iB* | 5,0 iB |
| Formel 66 | 2,0 bis 10 | 5,0 | 2,0 bis 10 P/B* | 5,0 P/B | 2,0 bis 10 iB* | 5,0 iB |
| Formel 67 | 2,0 bis 10 | 5,0 | 2,0 bis 10 P/B* | 5,0 P/B | 2,0 bis 10 iB* | 5,0 iB |
| Formel 68 | 2,0 bis 10 | 5,0 | 2,0 bis 10 P/B* | 5,0 P/B | 2,0 bis 10 iB* | 5,0 iB |
| Formel 69 | 2,0 bis 10 | 5,0 | 2,0 bis 10 P/B* | 5,0 P/B | 2,0 bis 10 iB* | 5,0 iB |
| Formel 70 | 2,0 bis 10 | 5,0 | 2,0 bis 10 P/B* | 5,0 P/B | 2,0 bis 10 iB* | 5,0 iB |
| Formel 71 | 2,0 bis 10 | 5,0 | 2,0 bis 10 P/B* | 5,0 P/B | 2,0 bis 10 iB* | 5,0 iB |
| Formel 72 | 2,0 bis 10 | 5,0 | 2,0 bis 10 P/B* | 5,0 P/B | 2,0 bis 10 iB* | 5,0 iB |
| Formel 73 | 2,0 bis 10 | 5,0 | 2,0 bis 10 P/B* | 5,0 P/B | 2,0 bis 10 iB* | 5,0 iB |
| Formel 74 | 2,0 bis 10 | 5,0 | 2,0 bis 10 P/B* | 5,0 P/B | 2,0 bis 10 iB* | 5,0 iB |
| Formel 75 | 2,0 bis 10 | 5,0 | 2,0 bis 10 P/B* | 5,0 P/B | 2,0 bis 10 iB* | 5,0 iB |
| * "P/B" entspricht einem Propan/Butan Gemisch<br>** "iB" entspricht Isobutan | | | | | | |

Wie eingangs ausgeführt, weisen die erfindungsgemäßen Mittel vorteilhafte haarfixierende Eigenschaften auf. Ein Verfahren zur temporären Verformung keratinischer Fasern, bei welchem eine erfindungsgemäße Zusammensetzung auf die keratinische Faser aufgebracht wird, ist daher ein weiterer Gegenstand der vorliegenden Anmeldung. Die Verwendung eines erfindungsgemäßen kosmetischen Mittels zur temporären Verformung keratinischer Fasern ist ein weiterer Gegenstand der vorliegenden Anmeldung. Wie eingangs ausgeführt, zeichnen sich die erfindungsgemäßen Mittel insbesondere durch einen verbesserten Halt bei der temporären Verformung keratinischer Fasern aus. Ein zusätzlicher Gegenstand der vorliegenden Anmeldung ist daher die Verwendung eines erfindungsgemäßen kosmetischen Mittels zur Verbesserung des Halts bei der temporären Verformung keratinischer Fasern.

Beispiele

Es wurde die Feuchtebeständigkeit (High Humidity Curl Retention; HHCR) der mittels der nachfolgenden drei haarkosmetischen Mittel erzielten temporären Haarverformung bestimmt.

| | **E1** | **V1** | **V2** |
|---|---|---|---|
| Fixomer A-30[1] | 6,25 | 12,5 | -- |
| Gafquat HS-100[2] | 3,75 | -- | 7,5 |
| Natriumbenzoat | 0,3 | 0,3 | 0,3 |
| D-Panthenol (75%) | 0,2 | 0,2 | 0,2 |
| Dow Corning 939[3] | 0,2 | 0,2 | 0,2 |
| Dehyquart A CA[4] | 1,0 | 1,0 | 1,0 |
| Castor Oil hydrogenated, 40 EO | 0,2 | 0,2 | 0,2 |

(fortgesetzt)

| | E1 | V1 | V2 |
|---|---|---|---|
| Parfüm | 0,1 | 0,1 | 0,1 |
| Wasser, Misc | add 100 | add 100 | add 100 |

[1] Copolymere von Methacrylsäure und 2-Acrylamido-2-methylpropansulfonsäure (12% in Wasser)
[2] Vinylpyrrolidon/Methacrylamidopropyltrimethylammoniumchlorid Copolymer (INCI: Polyquaternium-28, 20% in Wasser)
[3] Silikon Zubereitung (INCI: Amodimethicone, Trideceth-12, Cetrimonium Chloride)
[4] Trimethylhexadecylammoniumchlorid

Zur Bestimmung der High Humidity Curl Retention wurden standardisierte Haarsträhnen der Fa. Kerling (Art. Nr. 827560) des Haartyps "European Natural, Farbe 6/0) von einer Länge (Lmax) von 220 mm und einem Gewicht von 0.6 g eingesetzt. Zur Vorbereitung wurden die Strähnen mit einer 12.5 Gew.-%igen Natriumlaurethsulphat-Lösung gewaschen. Die Haarsträhnen wurden über Nacht in einem Trockenofen bei 318 K getrocknet.

0.18 g der Zusammensetzungen wurden auf jeweils eine Haarsträhne appliziert und einmassiert. Die Strähne wurde sodann auf einen Wickler gewickelt (Fripac-medis, ∅ 7mm, Art. Nr. D-1203) und über Nacht bei Raumtemperatur getrocknet.

Die Wickler wurden vorsichtig entfernt und die Strähne aufgehängt. Die Längen der Locken wurden jeweils gemessen ($L_0$) und die Strähnen in eine Klimakammer gegeben. Dort wurden sie bei 294 K und einer relativen Luftfeuchtigkeit von 85% über einen Zeitraum von 6 h gelagert und danach die Längen der Locken erneut vermessen (Lt).

Pro Zusammensetzung wurden 5 Teststrähnen entsprechend behandelt und vermessen.

Die High-Humidity-Curlretention (HHCR) wurde nach folgender Formel berechnet und für jede Zusammensetzung das arithmetische Mittel aus den HHCR-Werten der 5 Teststrähnen gebildet:

$$HHCR = \frac{L_{max} - L_t}{L_{max} - L_0}$$

| | E1 | V1 | V2 |
|---|---|---|---|
| HHCR | 85% | 77% | 38% |

Den Messdaten ist die synergistische Wirkung der erfindungsgemäßen Polymerkombination in Zusammensetzung E1 zu entnehmen.

## Patentansprüche

1. Kosmetisches Mittel, enthaltend in einem kosmetisch akzeptablen Träger

   a) mindestens ein Copolymer A der 2-Acrylamido-2-methylpropansulfonsäure mit einem oder mehreren Monomeren ausgewählt aus Acrylsäure und Methacrylsäure,
   b) mindestens eine polymere quartäre Ammoniumverbindung aus der Gruppe der Copolymere B von Vinylpyrrolidon mit Methacrylamidopropyltrimethylammoniumchlorid.

2. Kosmetisches Mittel nach Anspruch 1, enthaltend in einem kosmetisch akzeptablen Träger mindestens ein Copolymer A der 2-Acrylamido-2-methylpropansulfonsäure mit Methacrylsäure.

3. Kosmetisches Mittel nach einem der vorherigen Ansprüche, **dadurch gekennzeichnet, dass** der Gewichtsanteil des Copolymers A am Gesamtgewicht des Mittels 0,05 bis 10 Gew.-%, vorzugsweise 0,1 bis 7,0 Gew.-% und insbesondere 0,2 bis 5,0 Gew.-% beträgt.

4. Kosmetisches Mittel nach einem der vorherigen Ansprüche, **dadurch gekennzeichnet, dass** der Gewichtsanteil des Copolymers B am Gesamtgewicht des Mittels 0,05 bis 10 Gew.-%, vorzugsweise 0,1 bis 7,0 Gew.-% und

insbesondere 0,2 bis 5,0 Gew.-% beträgt.

5. Kosmetisches Mittel nach einem der vorherigen Ansprüche, **dadurch gekennzeichnet, dass** das kosmetische Mittel weiterhin mindestens ein Alkanolamin, vorzugsweise 2-Amino-2-methylpropanol enthält.

6. Kosmetisches Mittel nach einem der vorherigen Ansprüche, **dadurch gekennzeichnet, dass** es weiterhin mindestens eine monomere quartäre Ammoniumverbindung, vorzugsweise eine monomere quartäre Ammoniumverbindung aus der Gruppe der Trimethylalkylammoniumhalogenide enthält.

7. Kosmetisches Mittel nach einem der vorherigen Ansprüche, **dadurch gekennzeichnet, dass** es weiterhin mindestens einen Ölkörper, vorzugsweise mindestens einen Ölkörper aus der Gruppe der Silikonöle, enthält.

8. Verwendung eines kosmetischen Mittels nach einem der vorherigen Ansprüche, zur temporären Verformung keratinischer Fasern.

9. Verfahren zur temporären Verformung keratinischer Fasern, bei welchem eine Zusammensetzung nach einem der Ansprüche 1 bis 7 auf die keratinische Faser aufgebracht wird.

## Claims

1. A cosmetic agent containing, in a cosmetically acceptable carrier,

   a) at least one copolymer A of 2-acrylamido-2-methylpropane sulfonic acid comprising one or more monomers selected from acrylic acid and methacrylic acid,
   b) at least one polymeric quaternary ammonium compound from the group of copolymers B of vinylpyrrolidone comprising methacrylamido propyl trimethylammonium chloride.

2. The cosmetic agent according to claim 1, containing, in a cosmetically acceptable carrier, at least one copolymer A of 2-acrylamido-2-methylpropane sulfonic acid comprising methacrylic acid.

3. The cosmetic agent according to one of the preceding claims, **characterized in that** the proportion by weight of the copolymer A in the total weight of the agent is from 0.05 to 10 wt.%, preferably from 0.1 to 7.0 wt.% and in particular from 0.2 to 5.0 wt.%.

4. The cosmetic agent according to one of the preceding claims, **characterized in that** the proportion by weight of the copolymer B in the total weight of the agent is from 0.05 to 10 wt.%, preferably from 0.1 to 7.0 wt.% and in particular from 0.2 to 5.0 wt.%.

5. The cosmetic agent according to one of the preceding claims, **characterized in that** the cosmetic agent also contains at least one alkanolamine, preferably 2-amino-2-methylpropanol.

6. The cosmetic agent according to one of the preceding claims, **characterized in that** the cosmetic agent also contains at least one monomeric quaternary ammonium compound, preferably a monomeric quaternary ammonium compound from the group of trimethylalkyl ammonium halides.

7. The cosmetic agent according to one of the preceding claims, **characterized in that** the cosmetic agent also contains at least one oil body, preferably at least one oil body from the group of silicone oils.

8. The use of a cosmetic agent according to one of the preceding claims for temporarily shaping keratin fibers.

9. A method for temporarily shaping keratin fibers in which a composition according to one of claims 1 to 7 is applied to the keratin fibers.

## Revendications

1. Produit cosmétique contenant, dans un support cosmétiquement acceptable :

a) au moins un copolymère A de l'acide 2-acrylamido-2-méthylpropansulfonique avec un ou plusieurs monomères choisis parmi l'acide acrylique et l'acide méthacrylique,
b) au moins un composé ammonium quaternaire polymère issu du groupe des copolymères B de vinylpyrrolidone avec du chlorure de méthacrylamidopropyltriméthylammonium.

**2.** Produit cosmétique selon la revendication 1 contenant, dans un support cosmétiquement acceptable au moins un copolymère A de l'acide 2-acrylamido-2-méthylpropansulfonique avec l'acide méthacrylique.

**3.** Produit cosmétique selon une des revendications précédentes, **caractérisé en ce que** la proportion pondérale du copolymère A rapportée au poids total du produit est 0,05 à 10 % en poids, de préférence 0,1 à 7,0 % en poids et en particulier 0,2 à 5,0 % en poids.

**4.** Produit cosmétique selon une des revendications précédentes, **caractérisé en ce que** la proportion pondérale du copolymère B rapportée au poids total du produit est 0,05 à 10 % en poids, de préférence 0,1 à 7,0 % en poids et en particulier 0,2 à 5,0 % en poids.

**5.** Produit cosmétique selon une des revendications précédentes, **caractérisé en ce que** le produit cosmétique contient en outre au moins une alcanolamine, de préférence le 2-amino-2-méthylpropanol.

**6.** Produit cosmétique selon une des revendications précédentes, **caractérisé en ce qu'**il contient en outre au moins un composé ammonium quaternaire monomère, de préférence un composé ammonium quaternaire monomère issu du groupe des halogénures de triméthylalkylammonium.

**7.** Produit cosmétique selon une des revendications précédentes, **caractérisé en ce qu'**il contient en outre au moins une huile, de préférence au moins une huile issue du groupe des huiles de silicone.

**8.** Utilisation d'un produit cosmétique selon une des revendications précédentes pour déformer temporairement des fibres de kératine.

**9.** Procédé de déformation temporaire de fibres de kératine, dans lequel on applique une composition selon une des revendications 1 à 7 sur les fibres de kératine.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- EP 2272500 A1